# EUROPEAN PATENT APPLICATION

(11) **EP 2 181 981 A1**
(43) Date of publication of application: **05.05.2010**
(21) Application number: 08018721.4
(22) Date of filing: 27.10.2008
(51) Int. Cl.: C07C 211/61, C07C 17/25, C07C 22/02, C07C 23/08, C07C 205/12

(54) **Process for the preparation of benzonorbornenes**

(71) Applicant: Syngenta Participations AG, 4058 Basel (CH)
(72) Inventor: Gribkov, Denis Syngenta Crop Protection Münchwilen AG, 4333 Münchwilen (CH); Antelmann, Björn Syngenta Crop Protection Münchwilen AG, 4333 Münchwilen (CH); Giordano, Fanny Syngenta Crop Protection Münchwilen AG, 4333 Münchwilen (CH); Walter, Harald Syngenta Crop Protection Münchwilen AG, 4332 Stein (CH)
(74) Representative: Hölscher, Ingo

(57) **Abstract**

The present invention relates to a novel a process for the preparation of 2,9-dichloromethylidene-5-amino-benzonorbornene which process comprises
a) reacting cyclopentadiene in the presence of a radical initiator and CXCl₃, wherein X is chloro or bromo, to a compound of formula II 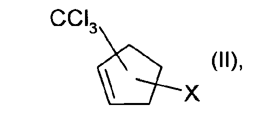 b) reacting the compound of formula II with a base in the presence of an appropriate solvent to the compound of formula III 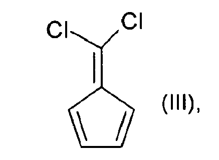 c) and converting the compound of formula II in the presence of 1,2-dehydro-6-nitrobenzene to the compound of formula IV 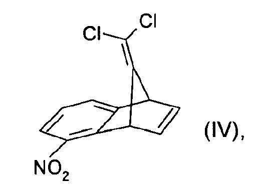 and d) hydrogenating the compound of formula IV in the presence of a metal catalyst.

## Description

The present invention relates to the preparation of 2,9-dichloromethylidene-5-amino-benzonorbornene.

The compound -52,9-dichloromethylidene-5-amino-benzonorbornene is a valuable intermediate for the preparation of benzonorbornene fungicides, as described for example in WO 2007/048556.

It is known from WO 2007/048556 to prepare 2,9-dichloromethylidene-5-amino-benzonorbornene by
a) reacting the compound of formula A 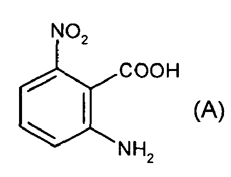 in the presence of an alkyl nitrite with a compound of formula B 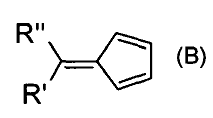 wherein R' and R" are e.g. C₁-C₄alkyl, to a compound of formula C 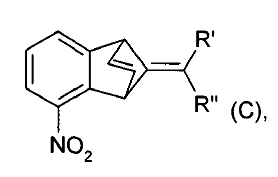
b) hydrogenating the compound of formula C in the presence of a suitable metal catalyst to a compound of formula D 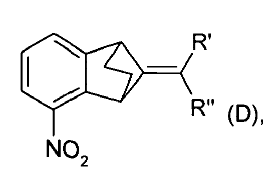
c) ozonising the compound of formula D with subsequent treatment with a reducing agent to a compound of formula E 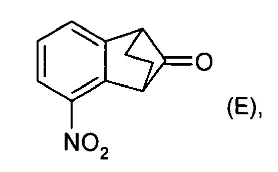
d) reacting the compound of formula E triphenylphosphine/carbon tetrachloride to 2,9-dichloromethylidene-5-nitro-benzonorbornene of formula F 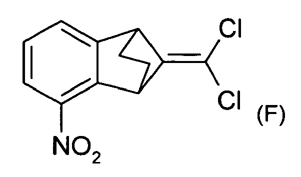 and
e) hydrogenating the compound of formula F in the presence of a metal catalyst to 2,9-dichloromethylidene-5-amino-benzonorbornene.

A disadvantage of this prior art process is the large number of reaction steps which decreases the yield of the product. In addition, the ozonolysis reaction which is difficult to handle and the expensive step d) which requires the use of triphenylphosphine makes this process uneconomic and unsuitable for a large-scale production.
The aim of the present invention is therefore to provide a novel process for the production of 2,9-dichloromethylidene-5-amino-benzonorbornene that avoids the disadvantages of the known process and makes it possible to prepare 2,9-dichloromethylidene-5-amino-benzonorbornene in high yields and good quality in an economically advantageous way with less reaction steps.

Thus, according to the present invention, there is provided a process for the preparation of 2,9-dichloromethylidene-5-amino-benzonorbornene of formula I 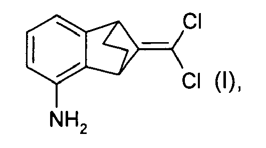 which process comprises
a) reacting cyclopentadiene with CXCl₃, wherein X is chloro or bromo; preferably bromo, in the presence of a radical initiator to a compound of formula II 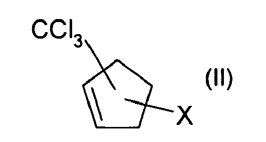 wherein X is chloro or bromo,
b) reacting the compound of formula II with a base in an appropriate solvent to the compound of formula III 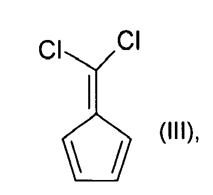
c) and converting the compound of formula III in the presence of 1,2-dehydro-6-nitrobenzene to the compound of formula IV 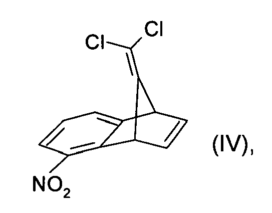 and
d) hydrogenating the compound of formula IV with a hydrogen source in the presence of a metal catalyst.

### Reaction step a):

The compound of formula II can occur in the following isomers or mixtures thereof: 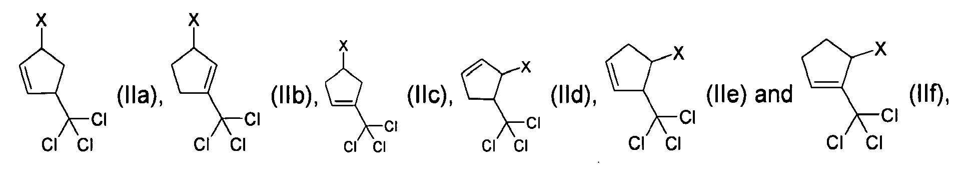 wherein X is chloro or bromo; preferably bromo.
The product of reaction step a) can be used as it is for the following reaction step b). The isolation or purification of a specific isomer or a isomer mixture of formula II is not necessary. The compound of formula II and its isomers and the compound of formula IV are novel and especially developed for the process according to the invention and therefore constitute a further object of the invention.

In principle a large number of radical initiators of following classes can be applied for reaction step a): organic peroxides (for example methyl ethyl ketone peroxide, benzoyl peroxide), organic azo compounds, metal salts and complexes (Cu, Ru). Preferred radical initiators are selected from azobisisobutyronitrile, dibenzoylperoxide and bis(tert-butylcyclohexyl)peroxydicarbonate. Especially preferred is azobisisobutyronitrile.

Reaction step a) is advantageously performed at elevated temperatures, preferably at temperatures of from 20 to 100°C, preferably of from 60 to 100 °C, most preferably of from 80 to 90°C. The use of bromotrichloromethane is preferred.

### Reaction step b):

Preferred bases for reaction step b) are alkali metal alcoholates, for example sodium tert-butoxide and potassium tert-butoxide or metal amides like NaNH₂ or lithiumdiisopropylamide. Except for ketones and esters, all inert solvents can be used. Appropriate solvents for reaction step b) are selected from methyl-tert-butylether (MTBE), methylcyclohexane (MCH) or a mixture thereof, tetrahydrofurane (THF), dyglime and toluene.

Reaction step b) is advantageously performed at a temperature range of -20 to +20°C, preferably at temperatures of from -10 °C to 10 °C, most preferably of from -5°C to 5°C.

In a preferred embodiment of the present invention the compound of formula III (its solution) is used without isolation directly for the next reaction step. The compound of formula III is a valuable intermediate for the preparation of the compound of formula I.The compound of formula III is known from (a) Moberg, C.; Nilsson, M. J. Organomet. Chem. 1973, 49, 243-248. (b) Siemionko, R. K.; Berson, J. A. J. Am. Chem. Soc. 1980, 102, 3870-3882.
However, the preparation method described in said reference is very unfavourable since:
1) The method could not be reproduced as described (low yield was obtained). 2) Utilizes very expensive pyrophoric and toxic starting material (nickelocene). 3) Produces large amount of metal-waste (nickel containing compounds). 4) The method cannot be scaled up for industrial use (unavailability of nickelocen in large amounts, dangerous handling of large quantities of nickelocen).

In contrast thereto, the preparation of the compound of formula III starting from cyclopentadiene is a novel and very efficient method and therefore constitutes a further object of the present invention.

Therefore, a further object of the present invention is a process for the preparation of the compound of formula III 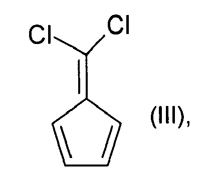 which process comprises
a) reacting cyclopentadiene in the presence of a radical initiator and CXCl₃, wherein X is chloro or bromo, preferably bromo,to a compound of formula II 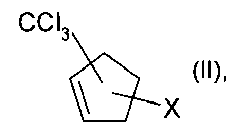 wherein X is chloro or bromo,
and b) reacting the compound of formula II with a base in the presence of an appropriate solvent.

### Reaction step c)

1,2-dehydro-6-nitrobenzene is generated *in situ* [for example, starting from a 6-nitroanthranilic acid of formula (A), 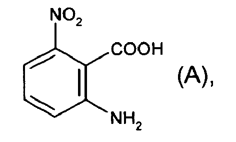 as described by L.Paquette et al, J. Amer. Chem. Soc. 99, 3734 (1977) and H. Seidel, Chemische Berichte, 34, 4351 (1901).

Reaction step c) is performed at elevated temperatures, preferably at temperatures of from 30°C to 60°C, most preferably of from 30 to 40°C.

### Reaction step d)

Preferred metal catalysts for the hydrogenation reaction are selected from the group consisting of Raney nickel, platinum, preferably platinum on a carrier like carbon, palladium, preferably palladium on a carrier like carbon but are not limited to said group. An especially preferred catalyst is Raney nickel. Suitable hydrogen sources are hydrogen or hydrazine, preferably hydrogen.
Reaction step d) is performed at low to elevated temperatures, preferably at temperatures of from 0 to 80 °C, preferably of from 30 to 60 °C.

### Preparatory examples:

### Example P1: Preparation of the compound of formula IIa:

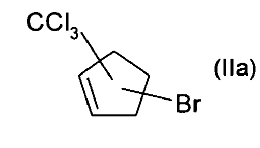

Azobisisobutyronitrile (2.5 g) was dissolved in bromotrichloromethane (250g).
Bromotrichloromethane (650 g) was loaded into a glass reactor under inert atmosphere (nitrogen) and heated to 85 °C. 1/3 of the azobisisobutyronitrile solution (84 g) was added into the reactor at once and the reactor content was heated again to 85 °C followed by simultaneous addition of the remaining 2/3 of azobisisobutyronitrile solution (168.5 g) and a mixture of cyclopentadiene (100 g, freshly distilled) and methylcyclohexane (10 g) during 2.5 hours at 85°C. The reaction mixture was stirred for an additional 1 hour at 85 °C, and then cooled to ambient temperature. Large amount of solvent (bromotrichloromethane) was evaporated in vacuum (60→70°C, 150→50 mbar). Methylcyclohexane (50 g) was added to the distillation residue and the distillation was continued (60→70°C, 150→15 mbar). The crude product (distillation residue) was dried in vacuum for an additional 30 min (70°C, 15 mbar). Yield 389 g of the compound of formula IIa in form of a brown oil, 94% pure, 92% yield, mixture of regioisomers.

### Example P2: Preparation of the compound of formula III:

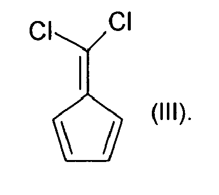

A glass reactor was loaded with bromo(trichloromethyl)cyclopentene (27.83 g, compound IIa), methylcyclohexane (62 mL), methyl-tert-butylether (62 mL) and bis(2-methoxyethyl) ether (diglyme, 6.7 g) under inert atmosphere (nitrogen). The mixture was cooled to -10 °C in an ice/NaCl bath. Sodium tert-butoxide (20.3 g) was added into the reactor as solid during 10 min while keeping the temperature below +5 °C. When the addition was done, the reaction mixture was stirred at 0-5 °C for 2 hours. The reaction mixture was quenched with a mixture of ice-cold water (80 mL) and ice (40 g) and then the pH value of the water phase was adjusted to ≤2 with 32% HCl (ca. 3 mL). The water phase was separated and the organic phase was dried over anhydrous potassium carbonate at 0°C. The potassium carbonate was filtered off and rinsed with methyl-tert-butylether (10 mL). Methyl ethyl ketone (10 g) was added to the combined filtrate as internal standard and the concentration of 6,6-dichlorofulvene (compound of formula III) was determined by ¹H NMR spectroscopy. Yield 81% [9.7 g of 9.9% (ca. 0.53 M) solution]. The solution was stored in a freezer and then used for the next step.

### Example P3: Preparation of the compound of formula IV:

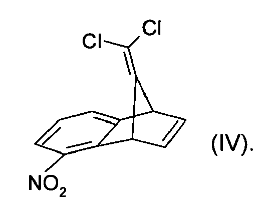

A cold solution of 6,6-dichlorofulvene obtained in the previous step (9% in methyl-tert-butylether/methylcyclohexane = 1:1) was placed in a glass reactor and heated quickly to 35°C. tert-pentyl nitrite (2.66 g) was added into the reactor followed by simultaneous addition of tert-pentyl nitrite (9.46 g) and a solution of 6-nitroanthranilic acid (11.6 g, 96.6%) in methyl ethyl ketone (42 mL) during 80 minutes at a temperature of 35°C. The reaction mixture was stirred for additional 30 min at the same temperature and then all the volatiles were removed by rotary evaporation. The remaining residue was crystallized from methanol (20 mL) at +5°C for 15 hours. The brown crystalline material was filtered, washed with cold methanol (15 mL) and dried in air. Yield 7.10 g (42%, 98% pure product).

### Example P4: Hydrogenation of the compound of formula IV:

An autoclave was charged with THF (130 ml), wet Raney-Nickel (2 g) and compound of formula IV (20 g). The autoclave was closed, the content started to agitate, purged three times with nitrogen to remove oxygen and then three times with hydrogen. The reactor was pressurized with hydrogen to 5 bar. Then the content of the autoclave was heated to 40 °C, maintaining pressure with additional hydrogen as needed. When hydrogen uptake stopped, typically after 3 -4 h, the reaction mass was held another 30 min at 40 °C. After this time, the pressure was released and the content was cooled to ambient temperature. The solvent was removed under vacuum and the resulting oil crystallized upon standing to yield 18 g of the yellowish to brownish compound of formula I (96 %, 94 % pure product).

## Claims

1. A process for the preparation of 2,9-dichloromethylidene-5-amino-benzonorbornene of formula I 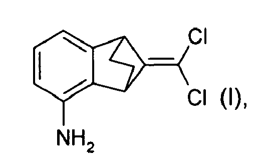 which process comprises
a) reacting cyclopentadiene with CXCl₃, wherein X is chloro or bromo, in the presence of a radical initiator to a compound of formula II 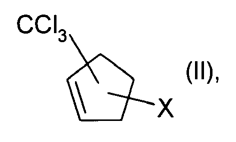 wherein X is chloro or bromo,
b) reacting the compound of formula II with a base in the presence of an appropriate solvent to the compound of formula III 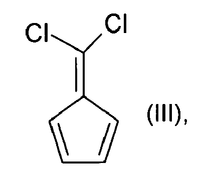
c) and converting the compound of formula III in the presence of 1,2-dehydro-6-nitrobenzene to the compound of formula IV 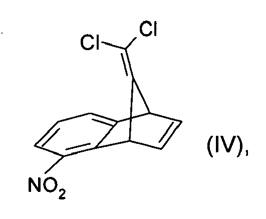 and
d) hydrogenating the compound of formula IV with a hydrogen source in the presence of a metal catalyst.

2. A compound of formula II 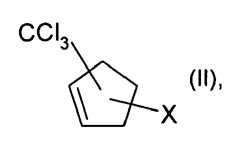 wherein X is chloro or bromo.

3. The compound of formula IV

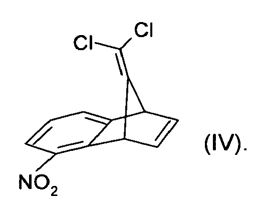

4. A process for the preparation of the compound of formula III 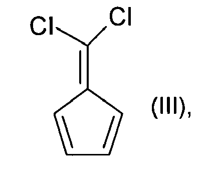 which process comprises
a) reacting cyclopentadiene in the presence of a radical initiator and CXCl₃, wherein X is chloro or bromo,to a compound of formula II 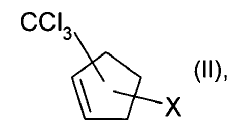 wherein X is chloro or bromo,
and b) reacting the compound of formula II with a base in the presence of an appropriate solvent.

5. A process according to claim 4, wherein X is bromo.

## Amended claims

### Amended claims in accordance with Rule 137(2) EPC.

**1.** A process for the preparation of 2,9-dichloromethylidene-5-amino-benzonorbornene of formula I 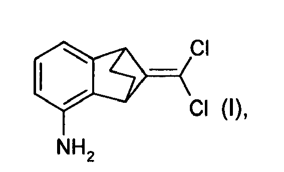 which process comprises
a) reacting cyclopentadiene with CXCl₃, wherein X is chloro or bromo, in the presence of a radical initiator to a compound of formula II 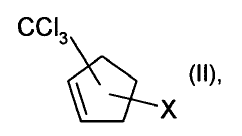 wherein X is chloro or bromo,
b) reacting the compound of formula II with a base in the presence of an appropriate solvent to the compound of formula III 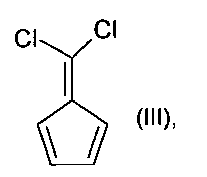
c) and converting the compound of formula III in the presence of 1,2-dehydro-6-nitrobenzene to the compound of formula IV 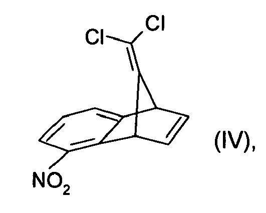 and
d) hydrogenating the compound of formula IV with a hydrogen source in the presence of a metal catalyst.

**2.** The compound of formula IV

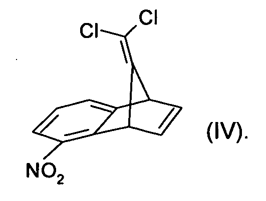

**3.** A process for the preparation of the compound of formula III 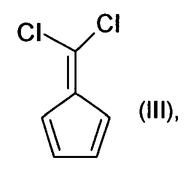 which process comprises
a) reacting cyclopentadiene in the presence of a radical initiator and CXCl₃, wherein X is chloro or bromo, to a compound of formula II 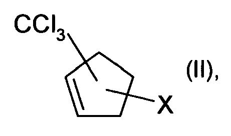 wherein X is chloro or bromo,
and b) reacting the compound of formula II with a base in the presence of an appropriate solvent.

**4.** A process according to claim 3, wherein X is bromo.
